# EUROPEAN PATENT APPLICATION

(11) **EP 4 417 223 A1**
(43) Date of publication of application: **21.08.2024**
(21) Application number: 23156714.0
(22) Date of filing: 15.02.2023
(51) Int. Cl.: A61L 2/18, A61B 90/70

(54) **A MEDICAL WASHER-DISINFECTOR**

(71) Applicant: Getinge Disinfection AB, 351 15 Växjö (SE)
(72) Inventor: Carl-Johan, LAGERKVIST, 352 20 Växjö (SE)
(74) Representative: Zacco Sweden AB

(57) **Abstract**

There is disclosed a medical washer-disinfector formed as a tunnel, comprising at least a liquid processing chamber and a drying chamber. A sealing door is provided which, in a closed position seals, off the liquid processing chamber from the drying chamber. A reuse tank is configured to receive water that has previously been discharged from a clean water tank. Each medical article is subjected to a post rinse step using the received water in the reuse tank, a final rinse step following the post rinse step and using clean water from the clean water tank, wherein the clean water that is used in the final rinse step has not been previously used in any one of said plurality of processing steps, a drying step following directly after the final rinse step. The post rinse step and the final rinse step are performed in the liquid processing chamber. The drying step is performed in the drying chamber.

## Description

### TECHNICAL FIELD

The present disclosure relates to a medical washer-disinfector. In particular, the present disclosure relates to a medical washer-disinfector formed as a tunnel having an inlet end for receiving medical articles to be washed and/or disinfected, and an opposite outlet end for discharging medical articles that have been moved through the tunnel.

### BACKGROUND ART

Medical washer-disinfectors may typically be provided in hospitals, such as at central sterile supply departments, and similar facilities. Medical washer-disinfectors may be used for washing and/or disinfecting a wide range of different medical articles, such as recirculated goods, tubular instruments, surgical sets, anaesthetic items, laboratory items, bedpans, etc.

Medical washer-disinfectors are commonly intended to provide both cleaning of visible physical contaminants from medical devices or medical equipment, as well as a degree of disinfection, such as by hot water exposure and/or cleaning/disinfection agents. Washing/disinfection processes for each batch of goods commonly include more than one phase. For example, they can include some or all of pre-rinse cycles, one or more washing cycles with various detergents and cleaning agents, one or more rinses, and/or a hot "thermal rinse" for disinfection.

In order to increase effectivity, some medical washer-disinfectors are formed as a tunnel having multiple chambers through which the medical articles are passed as they are subjected to different phases. In this manner, several batches of goods can be processed simultaneously, but in different chambers. While one batch of goods is being washed in one chamber, another batch of goods may be rinsed in another chamber, etc.

In some applications, the chambers will be drained at the end of each phase, and new liquid is added for the subsequent phase. Different phases can use different types of liquids, and different liquid circulation patterns. Conventional medical washer-disinfectors consume large amounts of water. Such consumption is neither desirable from an environmental perspective, nor from energy and economical perspectives. It would be desirable to maintain the effectivity of tunnel type medical washer-disinfectors, while reducing the water, energy, and chemical consumption.

### SUMMARY OF THE DISCLOSURE

An object of the present disclosure is to mitigate drawbacks of conventional washer-disinfectors. This and other objects, which will become apparent in the following, are accomplished by a medical washer-disinfector as defined in claim 1. Some non-limiting exemplary embodiments are presented in the dependent claims.

The disclosure includes the realization that the same load of water may be reused multiple times in particular advantageous sequences and manners, each time in a different process step and for a different batch of goods.

According to at least a first aspect of the present disclosure, there is provided a medical washer-disinfector formed as a tunnel having an inlet end for receiving medical articles to be washed and/or disinfected, and an opposite outlet end for discharging medical articles that have been moved through the tunnel, the medical washer-disinfector comprising:
- a plurality of chambers comprising at least one liquid processing chamber and a drying chamber, the drying chamber being located closest to said outlet end and downstream of the liquid processing chamber with respect to the direction of movement of the medical articles through the tunnel,
- a sealing door which in an open position allows medical articles to be moved from the liquid processing chamber to the drying chamber, and which in a closed position seals off the liquid processing chamber from the drying chamber,
- a clean water tank,
- a reuse tank configured to receive water that has previously been discharged from the clean water tank, in particular for receiving water after having been used in a liquid processing chamber,
wherein the medical washer-disinfector is configured to subject each medical article to a plurality of processing steps, including:
- a wash step,
- a post rinse step following the wash step and using the received water in the reuse tank,
- a final rinse step following the post rinse step and using clean water from the clean water tank, wherein the clean water that is used in the final rinse step has not been previously used in any one of said plurality of processing steps,
- a drying step following after the final rinse step, in particular directly after the final rinse step,
wherein the post rinse step and the final rinse step are performed in the liquid processing chamber, and the drying step is performed in the drying chamber.

In the embodiment above the washing step can take place in the liquid processing chamber, or can take place in a separate washing chamber (located upstream of the liquid processing chamber) when present.

From the above it can be understood that there are at least two different sequences followed in the present disclosure. One sequence concerns the order in which any medical article is subjected to the different process steps. As indicated above, the post rinse step is performed prior to the final rinse step, which in turn is performed prior to the drying step. Another sequence is the movement or usage of the water, which can be regarded as following a more or less reverse order (there is no water discharged in the drying step). The water that is first discharged in the final rinse step is then reused in a post rinse step. From these two different sequences carried out in the medical washer-disinfector, it can be understood that the clean water which is dispensed onto a first medical article in a final rinse step, can later be reused and dispensed onto a following second medical article in a post rinse step. The number of steps in each of the sequences may be extended. Thus, there may be a number of additional process steps, in particular there may be process steps performed prior to said post rinse step. Similarly, the water reused in the post rinse step may be reused one or more times in earlier steps. In this way, an article passing through the tunnel and chambers may become subjected to cleaner water for each process step. By allowing this reuse of water, an effective medically satisfactory washing and disinfecting can be achieved without too high water consumption. In particular embodiments, the water moves generally in the opposite direction from the goods: from the liquid processing chamber closest to the outlet end, towards the inlet end.

The above example of one medical article following another in the tunnel is at least partly reflected in the following exemplary embodiment. Thus, according to at least one exemplary embodiment, after a first medical article has been subjected to the final rinse step, the medical washer-disinfector is configured to move the first medical article to the drying chamber and to move a second medical article into the liquid processing chamber,
wherein the medical washer-disinfector is configured to collect water that has been used for rinsing the first medical article in the final rinse step and to store the collected water in the reuse tank,
wherein the medical washer-disinfector is configured to subsequently subject the second medical article to the post rinse step using said collected water stored in the reuse tank, and then subject the second medical article to the final rinse step using clean water from the clean water tank, and then move the second medical article to the drying chamber after or while removing the first medical article from the drying chamber,
in particular wherein the second medical article is subjected to the post rinse step and the final rinse step in the liquid processing chamber.

Thus, the movement of the medical articles can be appropriately synchronized with respect to the completion of the process steps, and thus with respect to the movement of the water from the clean water tank via the liquid processing chamber to the reuse tank.

When washing medical articles in a washer-disinfector the medical articles may be subjected to a detergent or other type of cleaning agent, which should be removed by rinsing. Suitably, there may be several rinse steps to make sure all or substantially all detergent has been removed. The final rinse step may suitable include subjecting the medical articles to water at a high temperature in order to achieve a disinfecting effect so as to deactivate microorganisms. However, before subjecting the medical article to such high temperature, there may be several rinse steps at lower temperature, having the primary function of removing detergents, residual material, etc. Such rinse steps performed after washing may be referred to as "post rinse steps". At least some exemplary embodiments of the present disclosure includes the use of two more such post rinse steps, as will be discussed in the following.

According to at least one exemplary embodiment, said reuse tank is a first reuse tank and said post rinse step using the received water in the reuse tank is a second post rinse step, wherein the medical washer-disinfector further comprises a second reuse tank configured to receive water that has previously been discharged from the first reuse tank, wherein said plurality of processing steps further includes:
- a first post rinse step using the received water in the second reuse tank,
wherein for any medical article the first post rinse step is performed in the liquid processing chamber, and is followed by the second post rinse step.

Thus, each one of the first post rinse step, second post rinse step and final post rinse step may be performed in the liquid processing chamber. However, the same batch of water will only be used once for each article in the liquid processing chamber. From the above it can also be understood that the processing sequence for an individual medical article is the first post rinse step, followed by the second post rinse step, followed by the final rinse step. The movement of the water is however in the reverse direction, i.e. first used in the final rinse step for a medical article, then reused in the second post rinse step for another medical article, and then reused in the first post rinse step for yet another medical article. This is at least partly reflected in the following exemplary embodiment.

According to at least one exemplary embodiment, for a series of three medical articles that are moved one after each other through the tunnel, the last one of the three medical articles is, in the first post rinse step, flushed with the same water that has earlier been used to flush the middle one of the three medical articles in the second post rinse step, which in turn is the same water that has even earlier been used to flush the first one of the three medical articles in the final rinse step.

Thus, the water may be reused multiple times, each time in a different process step and for a different medical article. In this way the water consumption can be reduced without compromising on efficiency and quality washing and/or disinfecting.

According to at least one exemplary embodiment, said plurality of chambers further comprises:
- a washing chamber located upstream of said liquid processing chamber with respect to the direction of movement of the medical articles through the tunnel,
wherein the medical washer-disinfector further comprises:
- a third reuse tank configured to receive water that has previously been discharged from the second reuse tank,
wherein for any medical article the wash step is performed in the washing chamber using the received water in the third reuse tank, and is performed prior to the post rinse steps.
The wash step may include adding detergent or other additive cleaning agent to water. For example, it may be added to the third reuse tank where it is mixed with the reused water or it may be dispensed separately into the washing chamber.

By having a separate washing chamber located upstream of the liquid processing chamber, one medical article may be subjected to the wash step in the washing chamber simultaneously with another medical article being subjected to one or more post rinse steps and/or final rinse step in the liquid processing chamber. In other exemplary embodiments, however, the wash step may be performed in the liquid processing chamber. Although such embodiments do not allow as many medical articles to be treated simultaneously as having a separate washing chamber, they have the advantage of forming a shorter tunnel, which may be desired in facilities having limited space for the medical washer-disinfector.

According to at least one exemplary embodiment, said plurality of processing steps further includes:
- a pre rinse step using water that has not been used in any other one of said plurality of processing steps,
wherein for any medical article the pre rinse step is performed prior to the other ones of said plurality of processing steps, in particular prior to at least the wash step, the post rinse step, the final rinse step and the drying step. For any embodiments including a wash step, a first post rinse step and/or a second post rinse step, the pre rinse step is performed prior to such step or steps. The pre rinse step may in particular use cold water. An advantage of avoiding reusing the water from the other processing steps is that blood tends to stick to surfaces at temperatures above 30°C - 40°C. Reused water may therefore be too warm for pre rinsing, in particular for the first pre rinsing if more the one pre rinsing steps are performed.

According to at least one exemplar embodiment, said pre rinse step is a first pre rinse step, wherein said plurality of processing steps further includes:
- a second pre rinse step,
wherein for any medical article the second pre rinse step is performed subsequent to the first pre rinse step but prior to the other ones of said plurality of processing steps, in particular prior to at least the wash step, the post rinse step, the final rinse step and the drying step. For any embodiments including a wash step, a first post rinse step and/or a second post rinse step, the second pre rinse step is performed prior to such step or steps.

According to at least one exemplary embodiment, said medical washer-disinfector further comprises a fourth reuse tank configured to receive water that has previously been dispensed from the third reuse tank, wherein said second pre rinse step includes using the received water in the fourth reuse tank and is performed in the washing chamber or in a rinse chamber located upstream of said washing chamber with respect to the direction of movement of the medical articles through the tunnel.

Thus, a batch of water may advantageously be reused at least four times, in two or even three different chambers. Similarly to the post rinse step or steps being performed in the same chamber as the final rinse step, i.e. in the liquid processing chamber, the one or more pre rinse steps may be performed in the same chamber as the wash step, i.e. in the washing chamber. However, in other exemplary embodiments, the washing chamber may be dedicated to the wash step, while a separate upstream rinse chamber is dedicated to the one or more pre rinse steps.

According to at least one exemplary embodiment, the medical washer-disinfector further comprises an ultrasonic chamber for subjecting medical articles to ultrasonic treatment, in particular wherein the ultrasonic chamber is located between the washing chamber and the liquid processing chamber with respect to the direction of movement of the medical articles through the tunnel.

Ultrasonic treatment is advantageous because it aids removing soil from the surfaces of the medical articles, including small and out-of-the-way surfaces which cannot easily be reached or seen. Ultrasonic treatment may, for instance, be performed by providing a medium, such as water with a detergent, in the ultrasonic chamber, wherein ultrasonic soundwaves are caused to implode microscopic bubbles in the medium at the surfaces of the medical articles, thereby removing soil. The medium may be reused in the ultrasonic chamber multiple times. For instance, the medium in the ultrasonic chamber may be exchanged once a day. The reused water bypasses the ultrasonic chamber, i.e. when a batch of water has been used for the last time in the liquid processing chamber, e.g. in a first post rinse step, it will be collected and fed to the wash chamber (or rather the third reuse tank associated with the wash chamber) located upstream of the ultrasonic chamber with respect to the direction of movement of the medical articles through the tunnel. From the above, it should be understood that in at least some exemplary embodiments, said plurality of chambers may comprise at least four chambers. For example, in some exemplary embodiments the medical washer-disinfector may present a tunnel formed by a washing chamber for one or more pre rinse steps and a wash step, followed by an ultrasonic chamber for said ultrasonic treatment, followed by a liquid processing chamber for one or more post rinse steps and a final rinse step, followed by a drying chamber for a drying step. In other exemplary embodiments, the medical washer-disinfector may present a tunnel formed by a rinse chamber for one or more pre rinse steps, followed by a washing chamber for a wash step, followed by a liquid processing chamber for one or more post rinse steps and a final rinse step, followed by a drying chamber for a drying step.

From the above, it can also be understood that in some exemplary embodiments the inlet end of the medical washer-disinfector may be presented by a wash chamber, while in other exemplary embodiments the inlet end may be presented by a rinse chamber. In exemplary embodiments having only two chambers, the inlet end may even be presented by a liquid processing chamber. In either one of the above examples, the outlet end may suitably be presented by a drying chamber.

According to at least one exemplary embodiment, the medical washer-disinfector is configured to heat the clean water in the clean water tank, wherein after the heated clean water has been dispensed from the clean water tank in a final rinse step, the heated water is reused multiple times via reuse tanks without additional heating, each time being reused on a different medical article. For instance, the clean water may suitably be heated to a temperature at which microorganisms are deactivated. The temperature of the water will naturally decrease over time, but may still have a sufficiently raised temperature throughout the various process steps so that the raised temperature of the water contributes in cleaning the medical article. As a purely illustrative example, the clean water in the water tank may, for instance, be heated to a temperature of 90°C or more, and may then be reused multiple times, maintaining a temperature of 50°C or more, before the reused water is disposed of through a drain.

According to at least one exemplary embodiment, the heated water is reused at least four times on at least four different batches of medical articles. Since the heated water has not been in any process step before the final rinse step, the total number of uses of the heated water may be at least five, i.e. as clean water in the use in the final rinse step, and the at least the four reuses. In other words, according to at least some exemplary embodiments, the heated water is used at least five times on at least five different medical articles. By reusing the heated water multiple times, not only is water consumption kept low, but also energy consumption may be kept low since there is no need to reheat the water.

From the above, it can also be understood that, in at least some exemplary embodiments, for each medical article passing through the tunnel, the medical washer-disinfector may be configured to subject the medical article to multiple different loads of water, in particular at least five different loads of water, and never the same load of water twice. The five different loads (or batches) of water may, for instance, be a first load in a pre rinse step, a second load in a wash step, a third load in first post rinse step, a fourth load in a second post rinse step, and a fifth load in a final rinse step.

From the above, it can also be understood that, the same load of water may be used at least twice in the liquid processing chamber, and then at least twice in the washing chamber. As previously explained, the one or more post rinse steps and the final rinse step may be performed in the liquid processing chamber, and the one or more pre rinse steps and the wash step may be performed in the washing chamber. It should be understood that various different distribution of the process steps in various chambers are conceivable. On the one hand it may be advantageous to have many chambers and performing one process step in each chamber, as that would allow many medical articles to be subjected to a respective process step, simultaneously. On the other hand by using one chamber for two or more process steps, fewer chambers are needed, and therefore the overall dimension of the medical washer-disinfector may be reduced. Thus, for any individual medical washer-disinfector to be installed in any given facility, the available space and the desired efficiency should be taken into account when balancing the desire of having an efficient medical washer-disinfector with the desire of having a sufficiently compact configuration. For instance, according to at least some exemplary embodiments, the same load of water may be used at least twice in the liquid processing chamber and then once in the washing chamber and then once in the rinse chamber.

According to at least one exemplary embodiment, the medical washer-disinfector further comprises:
- a transporting arrangement configured to move medical articles through the tunnel, and
- a control unit configured to control the execution of said plurality of processing steps and to control the transporting arrangement to move the medical articles based on completion of one or more of said plurality of processing steps.
Thus, the transportation of the medical articles through the various chambers and process steps may suitably be executed automatically through the control of the control unit. The transporting arrangement may include any suitable conveying arrangement, such as rolls, conveyor belts, pushers, etc. The control unit may suitably also be configured to control the operation of the sealing doors between neighboring chambers, so as to open and close the doors in a timely manner. The sealing doors may, for instance, be sliding doors. Furthermore, the control unit may suitably be configured to operate other components of the medical washer-disinfector, such as pumps for pumping collected water to reuse tanks, valves for discharging water from any tanks, nozzles and spray wings from dispensing water onto the medical articles, etc.

This disclosure includes electronics and electronic instructions for implementing the devices and processes described herein, which may be embodied as a control unit. The control unit may include a microprocessor, microcontroller, programmable digital signal processor, computer, or another programmable device. The control unit may also, or instead, include an application specific integrated circuit, a programmable gate array or programmable array logic, a programmable logic device, or a digital signal processor. Where it includes a programmable device such as the microprocessor, microcontroller or programmable digital signal processor mentioned above, the processor may further include computer executable code that controls operation of the programmable device. The control unit is preferably configured for use with medical washer-disinfectors, and provided with electronic instructions to perform and control the execution of the plurality of processing steps and the control of the transporting arrangement and other components.

According to at least a second aspect of the disclosure, there is provided a method for operating a medical washer-disinfector formed as a tunnel having an inlet end for receiving medical articles to be washed and/or disinfected, and an opposite outlet end for discharging medical articles that have been moved through the tunnel, the medical washer-disinfector comprising:
- a plurality of chambers comprising at least one liquid processing chamber and a drying chamber, the drying chamber being located closest to said outlet end and downstream of the liquid processing chamber with respect to the direction of movement of the medical articles through the tunnel,
- a sealing door which in an open position allows medical articles to be moved from the liquid processing chamber to the drying chamber, and which in a closed position seals off the liquid processing chamber from the drying chamber,

- a clean water tank,
- a reuse tank configured to receive water that has previously been discharged from the clean water tank,
the method comprising:
- subjecting each medical article to a plurality of processing steps, including
   - a wash step,
   - a post rinse step following the wash step by reusing the received water in the reuse tank,
   - a final rinse step following the post rinse step by using clean water from the clean water tank, wherein the clean water that is used in the final rinse step has not been previously used in any one of said plurality of processing steps,
   - a drying step following directly after the final rinse step,
wherein the method comprises performing the post rinse step and the final rinse step in the liquid processing chamber, and the drying step in the drying chamber.

The reader will understand that the various features and exemplary embodiments discussed in connection with the medical washer-disinfector of the first aspect may be readily implemented as corresponding exemplary embodiments of the method of the second aspect.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. Further features of, and advantages with, the present invention will become apparent when studying the appended claims and the following description. The skilled person realizes that different features of the present disclosure may be combined to create embodiments other than those described in the following, without departing from the scope of the disclosure.

### BRIEF DESCRIPTION OF THE DRAWINGS

Fig. 1 illustrates, in accordance with at least one exemplary embodiment of the present disclosure, a side view of a medical washer-disinfector.
Fig. 2 illustrates, in accordance with at least one exemplary embodiment of the present disclosure, a medical washer-disinfector in a perspective view, wherein some cover panels have been removed for making some interior parts visible.
Figs. 3-6 are schematic illustrations of a medical washer-disinfector according to at least some different exemplary embodiments of the present disclosure.

### DETAILED DESCRIPTION

The present disclosure will now be described more fully hereinafter with reference to the accompanying drawings, in which certain aspects of a medical washer-disinfector of the present disclosure are shown. The medical washer-disinfector may, however, be embodied in many different forms and should not be construed as limited to the embodiments and aspects set forth herein; rather, the embodiments are provided by way of example so that this disclosure will be thorough and complete, and will fully convey the scope of the medical washer-disinfector to those skilled in the art. Accordingly, it is to be understood that the present disclosure is not limited to the embodiments described herein and illustrated in the drawings; rather, the skilled person will recognize that many changes and modifications may be made within the scope of the appended claims. Like reference numerals refer to like elements throughout the description.

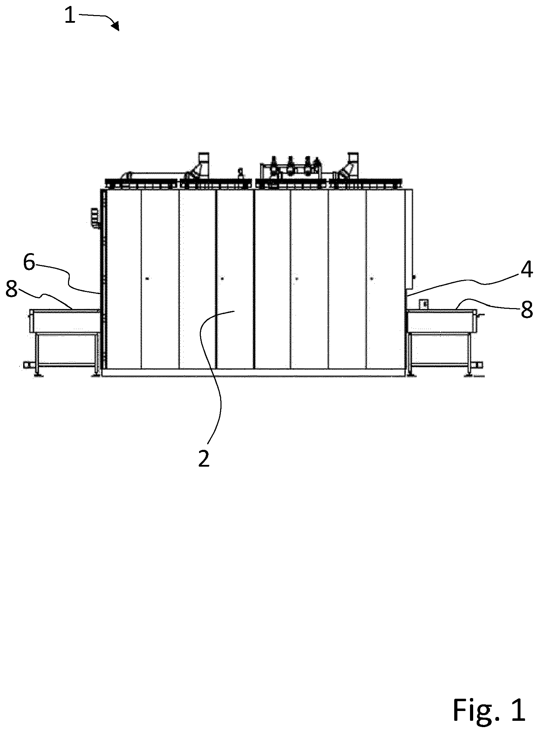

Fig. 1 illustrates, in accordance with at least one exemplary embodiment of the present disclosure, a side view of a medical washer-disinfector 1. The medical washer-disinfector 1 is formed as a tunnel 2 having an inlet end 4 for receiving medical articles to be washed and/or disinfected. The inlet end 4 may also be referred to as a load end. The medical washer-disinfector also has an opposite outlet end 6 for discharging medical articles that have been moved through the tunnel 2.

A transporting arrangement 8 may be provided for transporting the medical articles through the tunnel 2, from the inlet end 4 to the outlet end 6. The transporting arrangement 8 may, for instance, include a conveyor belt, rollers, or any other appropriate components and mechanisms for moving the medical articles from the inlet end 4, through the tunnel 2, to the outlet end 6. It should be understood that the movement of the medical articles through the tunnel 2 may suitably be made in stepwise movements, including pauses between the movements. Thus, the medical articles may, during a period of time, remain substantially stationary at one or more occasions during their travel through the tunnel 2, to allow sufficient time for processing/treatment of the medical articles during different processing steps.

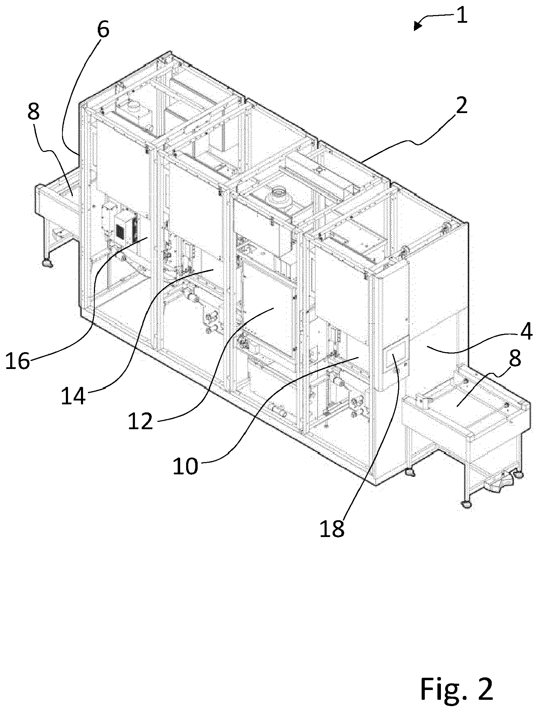

The processing/treatment of the medical articles is performed in different chambers. The medical washer-disinfector 1 comprises a plurality of chambers, i.e. at least two chambers. Such chambers can be better seen in Fig. 2. The medical washer-disinfector 1 shown in Fig. 2 may, for instance, be the same as the one shown in Fig. 1.

Fig. 2 illustrates, in accordance with at least one exemplary embodiment of the present disclosure, a medical washer-disinfector 1 in a perspective view, wherein some cover panels have been removed for making some interior parts visible. In the example of Fig. 2, the medical washer-disinfector 1 comprises four chambers 10, 12, 14, 16. Starting from the inlet end 2, the medical washer-disinfector 1 comprises a washing chamber 10, an ultrasonic chamber 12, a liquid processing chamber 14, and a drying chamber 16. It should, however, be understood that in other exemplary embodiments, the number and/or type of chambers may be different. For instance, in some exemplary embodiments, the ultrasonic chamber 12 may be omitted. In some exemplary embodiments, the washing chamber 10 as well as the ultrasonic chamber 12 may be omitted. As can be seen the tunnel 2 is formed by modules, each module housing one of said chambers 10, 12, 14, 16. Due to this modularity, the length of the tunnel 2, and the number and type of chambers can be conveniently adapted to the specific needs of any given facility, taking into account the available space and the type of treatment/processing that is desired to be provided to the medical articles.

The medical washer-disinfector 1 may comprise a user interface 18, such as a touch control panel, for displaying information and enabling a user to select a program. The user interface 18 may, for instance include different views, such as an overall view showing information simultaneously for the processes being performed in each chambers, such as remaining washing time, remaining, ultrasonic time, remaining rinsing time, remaining drying time, etc. Other views may show information specific to a certain chamber. Furthermore, the user interface 18 may show which load of medical article is present in which chamber, such as by presenting an identification number or code.

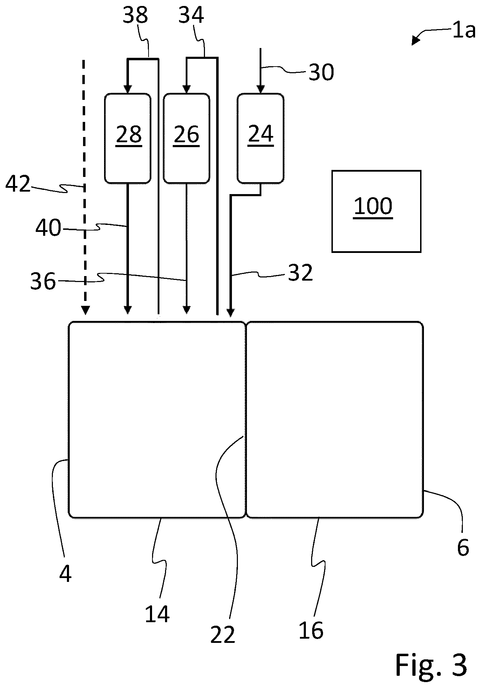

A more detailed discussion of the various chambers and the processes performed in the chambers will be discussed with reference to the schematic illustrations in Figs. 3-6. Before going into such details, an initial brief overview of each one of Figs. 3-6 will be presented.

Starting with Fig. 3, this is a schematic illustration of a medical washer-disinfector 1a according to at one exemplary embodiment of the present disclosure. In this example, the medical washer-disinfector 1a has only two chambers, namely a liquid processing chamber 14 and a drying chamber 16. In this and other embodiments, the single liquid processing chamber 14 can host many different liquid-based steps including pre-rinses, washes, post-rinses, and/or final rinses.

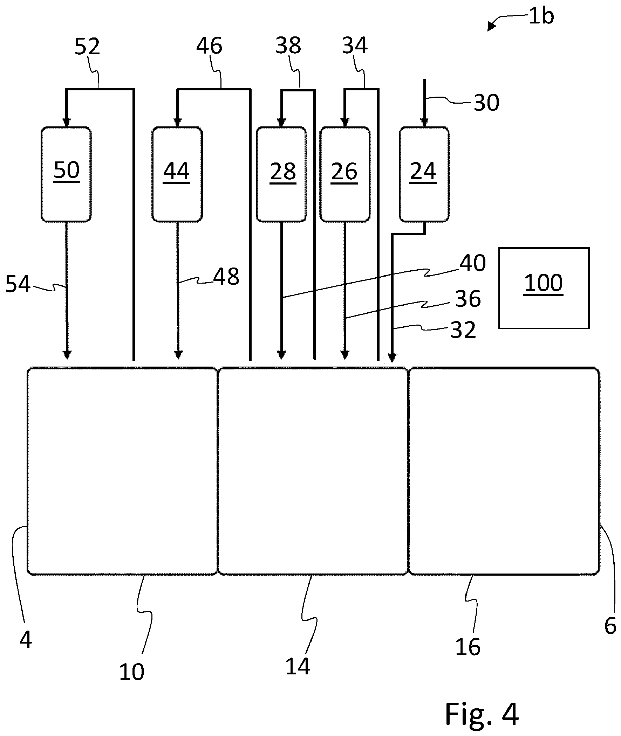

In Fig. 4, which is a schematic illustration of a medical washer-disinfector 1b according to at least another exemplary embodiment of the present disclosure, there are three chambers shown. In addition to the liquid processing chamber 14 and a drying chamber 16, there is also an additional separate washing chamber 10.

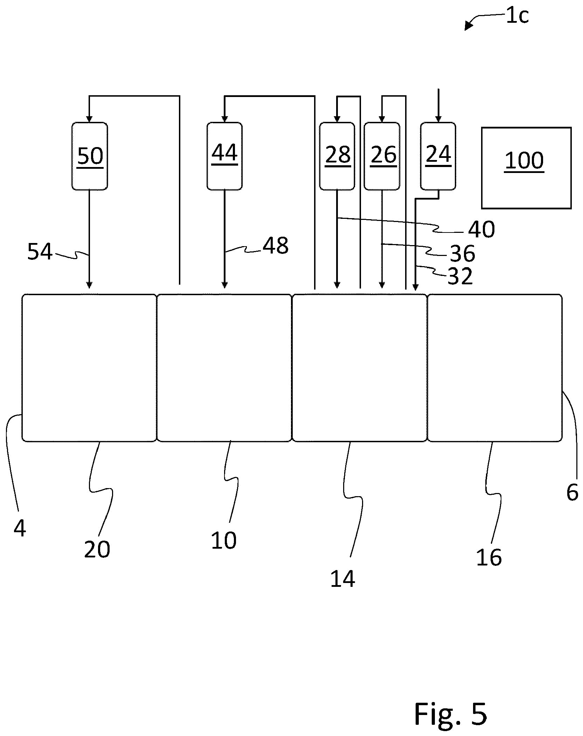

Fig. 5 is a schematic illustration of medical washer-disinfector 1c according to at least yet another exemplary embodiment of the present disclosure having four chambers. The last three chambers are, similarly to Fig 4, i.e. a washing chamber 10, a liquid processing chamber 14 and a drying chamber 16. In addition, there is also a separate rinse chamber 20 for pre-rinsing.

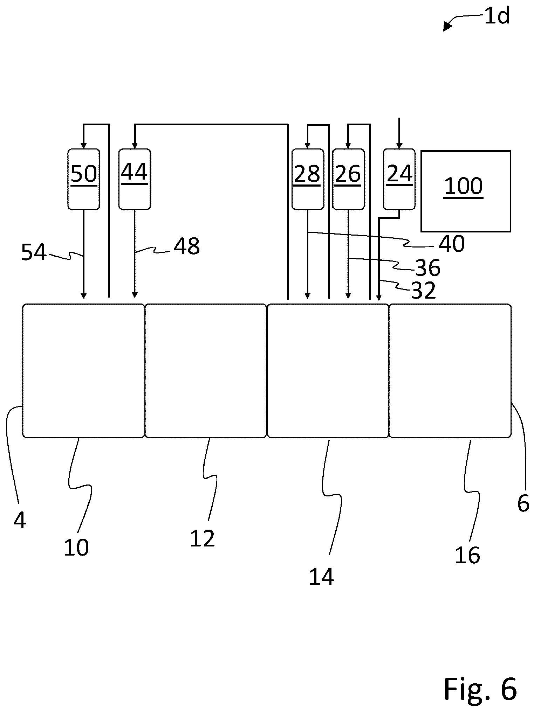

Fig. 6 is a schematic illustration of a medical washer-disinfector 1d according to at least a further exemplary embodiment of the present disclosure. The medical washer-disinfector 1d may, for example, correspond to the medical washer-disinfector 1 shown in Fig. 2, i.e. including a washing chamber 10, an ultrasonic chamber 12, a liquid processing chamber 14 and a drying chamber 16.

From the above it can be understood that each one of the schematic exemplary embodiments in Figs. 3-6 include a plurality of chambers comprising at least one liquid processing chamber 14 and a drying chamber 16, and that the drying chamber 16 is located closest to the outlet end 6 and downstream of the liquid processing chamber 14 with respect to the direction of movement of the medical articles through the tunnel.

Returning now to Fig. 3, for a more detailed discussion, the medical washer-disinfector 1a comprises a sealing door 22. The sealing door 22 is schematically represented by the dividing line between the chambers 14, 16. Similarly, in other exemplary embodiments having more than two chambers (such as those in Figs. 4-6), there may be a sealing door provided between any or all neighbouring chambers. In an open position of the sealing door 22, medical articles are allowed to move along the direction of movement through the tunnel (i.e. in the direction extending from the inlet end 4 to the outlet end 6). In the medical washer-disinfector shown in Fig. 3, in the open position of the sealing door 22, medical articles are allowed to move from the liquid processing chamber 14 to the drying chamber 16. In a closed position of the sealing door 22, it seals off the neighbouring chambers 14, 16 from each other, i.e. in the example of Fig. 3, it seals off the liquid processing chamber 14 from the drying chamber 16. In addition to having sealing doors 22 between any neighbouring chambers, there may suitably be provided a sealing door at the inlet end 4, i.e. at the entry into the first chamber (in Fig. 3 at the entry into the liquid processing chamber 14) and another sealing door at the outlet end 6, i.e. at the exit from the last chamber (in Fig. 3 at the exit from the drying chamber 16). The sealing doors reduce the risk of any cross-contamination between medical articles being processed in different chambers.

The medical washer-disinfector 1a also comprises a plurality of tanks for receiving, holding and discharging water. In the example of Fig. 3 there are three tanks 24, 26, 28 illustrated. In particular, the medical washer-disinfector comprises a clean water tank 24 which may receive clean water 30 that has not been previously used in any processing step in the medical washer-disinfector 1a. The clean water 30 may be provided from a general water supply system of the facility in which the medical washer-disinfector 1a has been installed. Water is discharged through appropriate passage(s) (including any nozzles, spray wings, etc.) from the clean water tank 24 into the liquid processing chamber 14 in a final rinse step 32. The final rinse step 32 is normally the last processing step in which the medical article is subjected to water. The water in the clean water tank 24 may suitably be heated to a temperature suitable for inactivating microorganisms. For instance, the water in the clean water tank 24 may be heated to 90°C.

The medical washer-disinfector 1a also comprises a reuse tank 26. The reuse tank 26 is configured to receive used water (as indicated by reference numeral 34) that has previously been discharged from the clean water tank 24 into the liquid processing chamber 14. Thus, the water is recovered from the liquid processing chamber 14 and is fed to the reuse tank 26. This recovered water in the reuse tank 26 may subsequently be used in a post rinse step 36 for rinsing another medical article which subsequently enters the liquid processing chamber 14 after the final rinse step 32 is completed for the medical article that is currently provided in the liquid processing chamber 14. One purpose of the post rinse step 36 may be to remove cleaning agents or detergents that have been used in previous wash steps.

From the previous discussion it can be understood that the medical washer-disinfector 1a is configured to subject each medical article to a plurality of processing steps, including a post rinse step 36 using the received water in the reuse tank 26 and a final rinse step 32 following the post rinse step 36 and using clean water from the clean water tank 24. Following these two steps the medical washer-disinfector 1a is configured to subject each medical article to another processing step, namely a drying step. As should now be understood, the post rinse step and the final rinse step are performed in the liquid processing chamber 14, and the drying step is performed in the drying chamber 16. The drying step may include heating the drying chamber and/or blowing air (such as filtered air) onto the medical article for efficiently drying the medical article.

In systems with fewer chambers, more steps could take place in each chamber. In the example of Fig. 3, the medical washer-disinfector 1a may be configured to perform a wash step in the liquid processing chamber 14, in addition to post-rinse and/or final rinse steps. In some embodiments there could also be one or more pre rinse steps before a wash step in the same liquid processing chamber or in a washing chamber.

The medical washer-disinfector 1a may suitably comprise a control unit 100 for controlling the operation of the medical washer-disinfector 1a, in particular the transportation of the medical articles through the tunnel and the execution of the various processing steps.

In order to not obscure the legibility of the drawings, the control unit 100 has consistently been illustrated as a separate component throughout the drawings. It should, however, be understood that the control unit 100 may be appropriately operatively connected to other components of the medical washer-disinfector, such as by wired or by wireless communication.

Continuing with Fig. 3, after a first medical article has been subjected to the final rinse step 32 in the liquid processing chamber 14, the medical washer-disinfector 1a may suitably move the first medical article to the drying chamber 16 and to move a second medical article into the liquid processing chamber 14. In particular, the control unit 100 may upon determination that the final rinse step 32 is completed, control the transporting arrangement (such as a transporting arrangement 8 illustrated in Figs. 1 and 2) to move the first medical article to the drying chamber 16 and to move the second medical article into the liquid processing chamber 14. The control unit 100 may also control the opening and closing of the sealing doors 22. The movement of the first medical article and second medical article may be made simultaneously, or suitably, the medical washer-disinfector 1a (in particular the control unit 100) may suspend the movement of the second medical article until the first medical article has been moved out from the liquid processing chamber 14. The medical washer-disinfector 1a may be configured to collect water that has been used for rinsing the first medical article in the liquid processing chamber 14 in the final rinse step 32 and to store the collected water in the reuse tank 26. Although not illustrated in the schematic illustration of Fig. 3, the liquid processing chamber 14 may be provided with a collectors, such as a collecting well at the bottom of the liquid processing chamber 14, from which a pump may feed the collected water through a conduit (as indicated by reference numeral 34) and through any valve into the reuse tank 26. The pump and valve may suitably be controlled by the control unit 100. The medical washer-disinfector 1a is configured to subsequently subject the second medical article to the post rinse step 36 using said collected water stored in the reuse tank 26, and then subject the second medical article to final rinse step 32 using clean water from the clean water tank 24. It should be understood that the second medical article will thus be subjected to the post rinse step 36 and the final rinse step 32 in the liquid processing chamber 14. After that, the second medical article can be moved to the drying chamber 16. This can be made after or while removing the first medical article from the drying chamber 16.

In addition to the clean water tank 24 and the reuse tank 26, there may be provided an additional reuse tank 28. The previously discussed reuse tank 26 may therefore be referred to as a first reuse tank 26, while the additional reuse tank 28 may be referred to as a second reuse tank 28. The second reuse tank 28 may be configured to receive water (as indicated by reference numeral 38) that has previously been discharged from the first reuse tank 26. Similarly to above, the control unit 100 may suitably control the pump and control any valve or valves to direct the collected water to the second reuse tank 28. For instance, there may be provided a valve for each reuse tank 26, 28, wherein the control unit 100 may control the opening and closing of the valves to direct the collected water to the desired reuse tank.

By being provided with the second reuse tank 28, the medical washer-disinfector 1a may perform several post rinse steps. In particular, there may be performed a first post rinse step 40 using water in the second reuse tank 28. Thus, for any medical article, the first post rinse step 40 would be performed in the liquid processing chamber 14, and would be followed by the second post rinse step 36.

It should be understood that the medical article will thus be subjected to three different batches of water during this three steps. In the first post rinse step 40 the medical article will be rinsed with a batch of water that has already been used twice on two previously processed medical articles in the liquid processing chamber 14 (i.e. the batch of water has already been used in the final rinse step 32 for one medical article, and then in the second post rinse step 36 for another medical article). When the first post rinse step 40 has been completed, the medical article will be subjected to the second post rinse step 36. In the second post rinse step 36 the medical article will be rinsed with a batch of water that has already been used once (i.e. the batch of water has already been used in the final rinse step 32 for said another medical article). When the second post rinse step 36 has been completed, the medical article will finally be subjected to the final rinse step 32 using a batch of water which has not been used before in any processing step in the chambers.

From the above it can be understood that for a series of three medical articles that are moved one after each other through the tunnel, the last one of the three medical articles is, in the first post rinse step 40, flushed with the same water that has earlier been used to flush the middle one of the three medical articles in the second post rinse step 36, which in turn is the same water that has even earlier been used to flush the first one of the three medical articles in the final rinse step 32. Thus, a single batch of water may be reused multiple times in different processing steps, and each time for a different medical article. In addition to this reuse of water, the medical washer-disinfector 1a may be configured to perform other processing steps in which the water is not necessarily reused. This illustrated by the dashed arrow 42. For example, the medical washer-disinfector 1a may be configured to perform a wash step in the liquid processing chamber 14, wherein said wash step does not use any reused water. Another example is that the medical washer-disinfector may be configured to perform one or more pre rinse steps before the wash step in the liquid processing chamber, for example to mechanically removing some material before using a detergent or other cleaning agent in the wash step. The dashed arrow 42 may also represent several processing steps performed without using any reused water, such as one or more pre rinse steps, as well as a wash step.

It should be understood that although reference has been made to "medical article" in singular, such as "a first medical article" and "a second medical article", each such medical articles may be processed together with other medical articles as a common load. For example, a plurality of medical articles may be provided on a tray, basket or other load carrying devices, wherein such load carrying devices are passed through the tunnel. The "medical article" may in practice be a rack or other structure holding multiple objects to be processed so that they move through the steps and chamber together.

The exemplary medical washer-disinfector 1a in Fig. 3 is very compact, having only two chambers 14, 16, and may therefore advantageously be installed in facilities having limited available space. However, the throughput is relatively low, as it can only hold two loads of medical articles at the same time. One load of medical article(s) may be present in the drying chamber 16 while another load of medical article(s) is present in the liquid processing chamber 14. However, the load of medical article(s) being present in the liquid processing chamber 14 may need to be subjected to a plurality of processing steps, such as any pre rinse step, any wash step, one or more post rinse steps and the final rinse step, before it can be moved to the drying chamber.

For embodiments having more chambers, the productivity will rise, as more loads of medical article(s) may be processed (subjected to different processing steps) simultaneously. Such examples will now be discussed in relation to Figs. 4-6.

In Fig. 4, there has been added a washing chamber 10, in addition to the liquid processing chamber 14 and the drying chamber 16. The washing chamber 10 is located upstream of the liquid processing chamber 14 with respect to the direction of movement of the medical articles through the tunnel. Furthermore, Fig. 4 illustrates that the medical washer-disinfector 1b comprises a third reuse tank 44 configured to receive water (as indicated by reference numeral 46) that has previously been discharged from the second reuse tank 28 into the liquid processing chamber 14. Suitably, the control unit 100 may be configured to control any valves and the pump to feed to the third reuse tank 44 the water that has been collected after discharge from the second reuse tank 28. Notably, the reuse of water in this case, is not only a reusing of water in another processing step, but also reusing of water in a different chamber. In this case, the water in the liquid processing chamber 14 will, time-wise, later be reused and discharged into the washing chamber 10. Thus, the medical washer-disinfector 1b may suitably be configured to perform a wash step 48 using the received water in the third reuse tank 44, wherein for any medical article the wash step 48 may be performed in the washing chamber 10 and prior to the post rinse steps 40, 36 and the final rinse step 32 (which are performed in the liquid processing chamber 14).

As illustrated in Fig. 4, there may be provided a fourth reuse tank 50 configured to receive water (as indicated by reference numeral 52) that has previously been dispensed from the third reuse tank 44. The fourth reuse tank 50 may suitably be used for a pre rinse step 54, i.e. before subjecting a medical article to the wash step 48 (in which chemical additives may be provided). Again, there may be provided a collector, such as a well, and the control unit 100 may control a pump to recover the collected water that has been dispensed from the third reuse tank 44 during washing of another medical article, in order to reuse the collected water when pre rinsing a subsequent medical article in the washing chamber 10. Suitably, if desired, the medical washer-disinfector may also be equipped with components for providing initial pre rinsing in the washing chamber with non-recovered water (similarly to what was discussed in connection with reference numeral 42 in Fig. 3). Such initial pre rinsing with non-recovered water may be performed in a first pre rinse step, whereas the water discharged from the fourth reuse tank 50 may be used in a second pre rinse step (i.e. pre rinse step 54).

Turning now to Fig. 5, which shows an exemplary embodiment of a medical washer-disinfector 1c having four chambers. The last three chambers are, similarly to Fig 4, a washing chamber 10, a liquid processing chamber 14 and a drying chamber 16. The first chamber is a rinse chamber 20. Thus, unlike the example in Fig. 4, in which the fourth reuse tank 50 is used for dispensing water in a pre rinse step 54 into the wash chamber 10, in the example in Fig. 5 the fourth reuse tank 50 is used for dispensing water in the pre rinse step 54 into a separate rinse chamber 20. This rinse chamber 20 is located upstream of the wash chamber 10 with respect to the direction of movement of the medical articles through the tunnel. In addition, there may be provided an optional pre rinse step performed prior to dispensing water from the fourth reuse tank 50. Such an optional pre rinse step may be performed by using water that has not been used in any other one of said plurality of processing steps 32, 36, 40, 48, 54. By having four chambers, four loads of medical articles can be subjected to respective processing steps simultaneously, each one in a different chamber.

Fig. 6 illustrates an example of a medical washer-disinfector 1d which also has four chambers. As mentioned previously, this example may, for example, correspond to the medical washer-disinfector 1 shown in Fig. 2, i.e. including a washing chamber 10, an ultrasonic chamber 12, a liquid processing chamber 14 and a drying chamber 16. The washing chamber 10 is at the inlet end 4. The drying chamber 16 is at the outlet end 6. When compared with the example of Fig. 4, the tanks 24, 26, 28, 44, 50 and the function of the chambers 10, 14 into which the water is dispensed may be substantially the same in the example of Fig. 6. Thus, in brief, water from the clean water tank 24 is discharged into the liquid processing chamber 14 in a final rinse step 32 on a first medical article. The water is then recovered to a first reuse tank 26 and is then discharged back into the liquid processing chamber 14 when a second post rinse step 36 is performed on a second medical article (the second medical article being upstream/behind the first medical article with respect to the movement of the medical articles through the tunnel). The water is then again recovered, this time to a second reuse tank 28, for reuse in a first post rinse step 40 when a third medical article has arrived into the liquid processing chamber 14. Then the water is recovered from the liquid processing chamber 14 and fed to a third reuse tank 44 for discharging water (including any additive) into the washing chamber 10 when performing a wash step 48. Then the water is again recovered to a fourth reuse tank 50 for performing a pre rinse step 54 in the washing chamber 10. Thus, the movement and recovery of water in the example of Fig. 6 substantially corresponds to the movement and recovery of water in the example of Fig. 4. However, in Fig. 6 an ultrasonic chamber 12 is provided for subjecting medical articles to ultrasonic treatment. The ultrasonic chamber 12 is located between the washing chamber 10 and the liquid processing chamber 14 with respect to the direction of movement of the medical articles through the tunnel. Because there are four chambers in the example in Fig. 6, four loads of medical articles can be treated simultaneously in a respective chamber. Similarly to what has been discussed in connection with the previous exemplary embodiments, the medical washer-disinfector 1d in Fig. 6 may, in addition to what is shown in the schematic illustration, start by performing an initial (first) pre rinse step, using water that has not been used before in any of the plurality of processing steps.

In each one of the illustrated examples, the medical washer-disinfector 1, 1a, 1b, 1c, 1d may suitably be configured to heat the clean water in the clean water tank 24. For instance, the control unit 100 may be configured to control heating elements. Suitably, the control unit 100 may receive feedback from one or more temperature sensors. After the heated clean water has been dispensed from the clean water tank 24 in the final rinse step 32, the heated water will be reused multiple times via the reuse tanks 26, 28, 44, 50 without additional heating, each time being reused on a different medical article. For instance, in the example in Fig. 3, the heated water can be reused two times (two reuse tanks 26, 28) on two different medical articles. In the examples in Figs. 4-6 the heated water can be reused four times (four reuse tanks 26, 28, 44, 50) on four different medical articles.

From the above discussion, it can be understood that for each medical article passing through the tunnel, the medical washer-disinfector 1, 1a, 1b, 1c, 1d may be configured to subject the medical article to multiple different loads of water. For instance, in the example in Fig. 3, apart from the three loads of water from the three tanks 24, 26, 28, each medial article may be subjected to separate loads of water in one or more pre rinse steps, and a wash step, each of these steps being performed with water that has not been used previously in any other processing steps. Thus, even in a medical washer having only two chambers (liquid processing chamber 14 and drying chamber 16), and only one of which is used for spraying or flushing water onto the medical articles (the liquid processing chamber 14), each medical article may be subject to at least five or even six different loads of water, and never the same load of water.

As illustrated in each one of the examples in Figs. 3-6, the same load of water may be used at least twice and even three times in the liquid processing chamber 14. As illustrated in the examples in Figs. 4 and 6, the same load of water may be used at least twice in the washing chamber 10. Thus, for the examples in Figs. 4 and 6, the same load of water is first used three times in the liquid processing chamber 14 and then used twice in the washing chamber 10. In Fig. 5, however, the same load of water is used twice in the liquid processing chamber 14 and then used one in the washing chamber 10 and then once in the rinse chamber 20.

It should be understood that, if space allows, in at least some exemplary embodiments, the medical-washer disinfector may have more than four chambers. For instance, each reuse tank may be configured to dispense the received water into a respective chamber.

From the above, it can be understood that in a general sense, according to at least some exemplary embodiments, the same load of water may be reused in at least one of said plurality of chambers.

This disclosure includes medical washer-disinfectors 1, 1a, 1b, 1c, 1d for processing medical articles to be washed and/or disinfected, and methods of operating such medical washer-disinfectors. In particular, this disclosure includes medical washer-disinfectors having a plurality of chambers 10, 12, 14,16, 20 forming a tunnel 2 through which medical articles are transported and presented to different processing steps 32, 36, 40, 48, 54. This disclosure also includes controllers (comprising electronics and electronic instructions) for controlling medical washer-disinfectors as described herein. The herein discussed control unit 100 is an example of such a controller. It should be understood that various features disclosed herein are contemplated and disclosed in their various combinations and sub-combinations.

## Claims

1. A medical washer-disinfector formed as a tunnel having an inlet end for receiving medical articles to be washed and/or disinfected, and an opposite outlet end for discharging medical articles that have been moved through the tunnel, the medical washer-disinfector comprising:
- a plurality of chambers comprising at least one liquid processing chamber and a drying chamber, the drying chamber being located closest to said outlet end and downstream of the liquid processing chamber with respect to the direction of movement of the medical articles through the tunnel,
- a sealing door which in an open position allows medical articles to be moved from the liquid processing chamber to the drying chamber, and which in a closed position seals off the liquid processing chamber from the drying chamber,
- a clean water tank,
- a reuse tank configured to receive water that has previously been discharged from the clean water tank,
wherein the medical washer-disinfector is configured to subject each medical article to a plurality of processing steps, including:
• a wash step,
• a post rinse step following the wash step and using the received water in the reuse tank,
• a final rinse step following the post rinse step and using clean water from the clean water tank, wherein the clean water that is used in the final rinse step has not been previously used in any one of said plurality of processing steps,
• a drying step following after the final rinse step, in particular directly after the final rinse step,
wherein the post rinse step and the final rinse step are performed in the liquid processing chamber, and the drying step is performed in the drying chamber.

2. The medical washer-disinfector according to claim 1, wherein
after a first medical article has been subjected to the final rinse step, the medical washer-disinfector is configured to move the first medical article to the drying chamber and to move a second medical article into the liquid processing chamber,
wherein the medical washer-disinfector is configured to collect water that has been used for rinsing the first medical article in the final rinse step and to store the collected water in the reuse tank,
wherein the medical washer-disinfector is configured to subsequently subject the second medical article to the post rinse step using said collected water stored in the reuse tank, and then subject the second medical article to the final rinse step using clean water from the clean water tank, and then move the second medical article to the drying chamber after or while removing the first medical article from the drying chamber,
in particular wherein the second medical article is subjected to the post rinse step and the final rinse step in the liquid processing chamber.

3. The medical washer-disinfector according to any one of claims 1-2, wherein said reuse tank is a first reuse tank and said post rinse step using the received water in the reuse tank is a second post rinse step, wherein the medical washer-disinfector further comprises a second reuse tank configured to receive water that has previously been discharged from the first reuse tank, wherein said plurality of processing steps further includes:
• a first post rinse step using the received water in the second reuse tank,
wherein for any medical article the first post rinse step is performed in the liquid processing chamber, and is followed by the second post rinse step.

4. The medical washer-disinfector according to claim 3, wherein for a series of three medical articles that are moved one after each other through the tunnel, the last one of the three medical articles is, in the first post rinse step, flushed with the same water that has earlier been used to flush the middle one of the three medical articles in the second post rinse step, which in turn is the same water that has even earlier been used to flush the first one of the three medical articles in the final rinse step.

5. The medical washer-disinfector according to any one of claims 3-4, said plurality of chambers further comprising:
- a washing chamber located upstream of said liquid processing chamber with respect to the direction of movement of the medical articles through the tunnel,
wherein the medical washer-disinfector further comprises:
- a third reuse tank configured to receive water that has previously been discharged from the second reuse tank,
wherein for any medical article the wash step is performed in the washing chamber using the received water in the third reuse tank, and is performed prior to the post rinse steps.

6. The medical washer-disinfector according to any one of claims 1-5, wherein said plurality of processing steps further includes:
• a pre rinse step using water that has not been used in any other one of said plurality of processing steps,
wherein for any medical article the pre rinse step is performed prior to the other ones of said plurality of processing steps, in particular prior to at least the wash step, the post rinse step, the final rinse step, and the drying step.

7. The medical washer-disinfector according to claim 6, wherein said pre rinse step is a first pre rinse step, wherein said plurality of processing steps further includes:
• a second pre rinse step,
wherein for any medical article the second pre rinse step is performed subsequent to the first pre rinse step but prior to the other ones of said plurality of processing steps, in particular prior to at least the wash step, the post rinse step, the final rinse step and the drying step.

8. The medical washer-disinfector according to claim 7 when dependent on claim 5, wherein said medical washer-disinfector further comprises a fourth reuse tank configured to receive water that has previously been dispensed from the third reuse tank, wherein said second pre rinse step includes using the received water in the fourth reuse tank and is performed in the washing chamber or in a rinse chamber located upstream of said washing chamber with respect to the direction of movement of the medical articles through the tunnel.

9. The medical washer-disinfector according to claim 5 or any one of claims 6-8 when dependent on claim 5, further comprising an ultrasonic chamber for subjecting medical articles to ultrasonic treatment, wherein the ultrasonic chamber is located between the washing chamber and the liquid processing chamber with respect to the direction of movement of the medical articles through the tunnel.

10. The medical washer-disinfector according to any one of claims 1-9, wherein the medical washer-disinfector is configured to heat the clean water in the clean water tank, wherein after the heated clean water has been dispensed from the clean water tank in a final rinse step, the heated water is reused multiple times via reuse tanks substantially without additional heating, each time being reused on a different medical article.

11. The medical washer-disinfector according to claim 10, wherein the heated water is used at least five times on at least five different medical articles.

12. The medical washer-disinfector according to any one of claims 1-11, wherein for each medical article passing through the tunnel, the medical washer-disinfector is configured to subject the medical article to multiple different loads of water, in particular at least five different loads of water, and never the same load of water twice.

13. The medical washer-disinfector according to claim 5 or any one of claims 6-12 when dependent on claim 5, wherein the same load of water is used at least twice in the liquid processing chamber, and then at least twice in the washing chamber.

14. The medical washer-disinfector according to claim 8 or any one of claims 9-12 when dependent on claim 8, wherein the same load of water is used at least twice in the liquid processing chamber and then once in the washing chamber and then once in the rinse chamber.

15. The medical washer-disinfector according to any one of claims 1-14, further comprising:
- a transporting arrangement configured to move medical articles through the tunnel, and
- a control unit configured to control the execution of said plurality of processing steps and to control the transporting arrangement to move the medical articles based on completion of one or more of said plurality of processing steps.
